(19) Europäisches Patentamt
European Patent Office
Office européen des brevets

(11) EP 0 458 064 B1

(12) EUROPEAN PATENT SPECIFICATION

(45) Date of publication and mention of the grant of the patent:
**25.02.1998 Bulletin 1998/09**

(51) Int Cl.[6]: **C07K 17/08**, A61K 47/48, C07C 233/31, C07C 237/22, C08G 65/32

(21) Application number: **91106224.8**

(22) Date of filing: **18.04.1991**

# (54) Stabilization of somatotropins by modification of cysteine residues

Stabilisierung von Somatotropin durch Modifizierung von Cysteinsresten

Stabilisation de la somatotropine à travers une modification des résidus de cystéine

(84) Designated Contracting States:
**AT BE CH DE DK ES FR GB GR IT LI LU NL SE**

(30) Priority: **04.05.1990 US 519047**

(43) Date of publication of application:
**27.11.1991 Bulletin 1991/48**

(73) Proprietor: **AMERICAN CYANAMID COMPANY**
**Wayne, NJ 07470-8426 (US)**

(72) Inventors:
- **Buckwalter, Brian Lee**
  **Yardley, Pennsylvania (US)**
- **Cady, Susan Mancini**
  **Yardley, Pennsylvania (US)**
- **Daley, Michael Joseph**
  **Yardley, Pennsylvania (US)**
- **Shieh, Hong-Ming**
  **Langhorne, Pennsylvania (US)**

(74) Representative:
**Wächtershäuser, Günter, Prof. Dr. et al**
**Patentanwalt,**
**Tal 29**
**80331 München (DE)**

(56) References cited:
**EP-A- 0 236 987** **EP-A- 0 355 460**
**WO-A-89/06546** **WO-A-90/12874**

- **J. BIOL. CHEM., vol. 263, no. 34, 5th December 1988, pages 18452-18458; G.M. FOX et al.: "Production, biological activity, and structure of recombinant basic fibroblast growth factor and an analog with cysteine replaced by serine"**
- **JOURNAL OF CONTROLLED RELEASE, vol. 11, nos. 1/3, January 1990, pages 139-148, Amsterdam, NL; F. FUERTGES et al.: "The clinical efficacy of poly(ethylene glycol)-modified proteins"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## BACKGROUND OF THE INVENTION

The present invention relates to novel derivatized recombinant or natural somatotropins wherein if said somatotropins contains one or more cysteine residue, said residue is derivatized with substituents as herein defined.

Although molecular biological techniques have dramatically increased the availability of many proteins and/or polypeptides (hereinafter referred to as proteins), the therapeutic use of said proteins is often times hindered by other factors, such as aggregation, proteolytic degradation, poor bioavailability and physical properties which preclude efficient formulations. In the present invention the highly reactive sulfhydryl groups on cysteine residues within both recombinantly-derived and naturally-derived proteins are derivatized to modify the effective charge on the protein surface, and thereby the isoelectric point, or to link a surface-modifying polymer to the protein.

The advantage of modifying the charge isoelectric point is well known due to the minimization of protein aggregation if the pH of a protein formulation is significantly different than the isoelectric point. Manipulation of the isoelectric point, therefore, affords a technique to minimize aggregation.

Also, another mechanism for enhancing protein formulation is by conjugation of the protein with derivatizing compounds, which include, but are not limited to, polyethylene glycol and polypropylene glycol. Some of these benefits recognized include: lowered immunogenicity and antigenicity, increased duration of action, and altered pharmacokinetic properties. [F.M. Veronese, Chimicaoggi (1989) 53].

Heretofore, attachment of polymeric molecules to proteins was principally through lysine or other basic amino acids. This was accomplished with alkylating agents such as triazine derivatives, diazonium salts or active acylating agents such as succinic acid derivatives. These reactions were difficult to control and significant losses in biological activity occurred frequently due to reactions with essential amino acids in the proteins [see F. F. Davis et al Polymer (1979) 20 357].

In contrast, the present invention provides a selective method for modifying the less abundant cysteine residues either naturally occurring within a protein or incorporated within a protein by such methods as site specific mutagenesis.

It is an object of the present invention to provide compositions of derivatized somatotropins biologically effective and yet stable to administer. It is another object of the present invention to provide compositions of biologically active recombinant and/or natural somatotropins which are suitable for parenteral administration, said compositions comprising a biologically active amount of a derivatized somatotropin (animal or human form) or pharmaceutically and pharmacologically acceptable salt thereof in a pharmaceutically and pharmacologically acceptable solid or liquid carrier.

It is a further object of the present invention to provide novel compositions which will allow the modification of any cysteine-containing proteins. By judicious selection of the nature and number of ionizable-functional groups and/or the size and nature of the surface-modifying polymer a range of derivatized proteins with distinctly different physical properties are produced.

These and other objects of the invention will become more apparent by the more detailed description of the invention provided hereinbelow.

## SUMMARY OF THE INVENTION

The present invention relates to novel derivatized recombinant or natural somatotropins wherein at least one-naturally-occurring or incorporated cysteine residue within the protein is derivatized with substituents selected from ($CH_2CO_2R_1$), ($CH_2CONHR_1$), [$CH(COR_2)(CH_2)_x(COR_3)$], [$CH_2$-$CONHCH(COR_2)(CH_2)_x(COR_3)$], or [$CH_2CONH(CH_2)_xCOR_4$]; wherein $R_1$ is $CH_2CH_2$-$(OCH_2CH_2)_yOMe$; $R_2$ and $R_3$ are H or $R_4$; $R_4$ is $NHCH_2CH_2(OCH_2CH_2)_yOMe$ or $OCH_2CH_2(OCH_2CH_2)_yOMe$; X is an integer of from 1 to 3 and Y is an integer of from 10 to 300; with the proviso that $R_2$ and $R_3$ cannot simultaneously be H; provided that if the derivatized cysteine residue(s) of said protein formed a disulfide linkage prior to derivatization, both cysteines will bear the same sulfhydryl derivatives after the derivatization procedure is complete. Furthermore, with some proteins containing more than one disulfide bond, it is possible to selectively cleave one or more of said disulfide bonds and those disulfide bonds which are selectively cleaved may be derivatized without affecting the remaining cysteine residues. When disulfides sequentially are cleaved it is possible to derivatize different cysteines with more than one composition. Furthermore, when said protein possesses an unpaired cysteine, that residues is derivatized without a reductive step.

This invention further relates to a method for inhibiting aggregation of a recombinant animal somatotropin, by reducing at least one of the two disulfide bridges between the cysteine amino acid residues at the 55 and 166 positions or at the 183 and 191 positions of said somatotropins and thereafter derivatizing each of the cysteines of the reduced bridge or bridges at the 55 and 166 positions and/or at the 183 and 191 positions with the same derivative, provided that the derivatives on the cysteines at the 55 and 166 positions are the same or different substituents than the substituents on the cysteines at the 183 and 191 positions. The substituents employed in the preparation of the novel

derivatized recombinant animal somatotropins of this invention include the following: $(CH_2CO_2R_1)$, $(CH_2CONHR_1)[CH(COR_2)(CH_2)_x-(COR_3)]$, $[CH_2CONHCH(COR_2)(CH_2)_x(COR_3)]$, or $[CH_2CONH-(CH_2)_xCOR_4]$; wherein $R_1$ is $CH_2CH_2(OCH_2CH_2)_yOMe$; $R_2$ and $R_3$ are H or $R_4$; $R_4$ is $NHCH_2CH_2(OCH_2CH_2)_yOMe$ or $OCH_2CH_2-(OCH_2CH_2)_yOMe$; X is an integer of from 1 to 3 and Y is an integer of from 10 to 300; with the proviso that $R_2$ and $R_3$ can not simultaneously be H.

In accordance with the present invention, the preferred novel animal somatotropins are recombinant porcine, bovine, ovine, human, avian and fish somatotropins wherein the disulfide bridge in the small loop of the somatotropin is reduced and the cysteines at the 183 and 191 positions derivatized with a substituent selected from $(CH_2COR_1)$, $[CH_2(CO_2R_2)(CH_2)_x(COR_3]$, $[CH_2CONHCH(COR_2)(CH_2)_x(COR_3)]$, or $[CH_2CONH(CH_2)_xCOR_4]$; wherein $R_1$, $R_2$, $R_3$, and X are as described.

All of the plasmids, DNA sequences and microorganisms deposited in connection with the present patent application, except where specified to the contrary, are deposited in American Cyanamid Company culture collection maintained in Princeton, New Jersey and are deposited with the American Type Culture Collection (ATCC) in Rockville, Maryland 20952, U.S.A.

## DETAILED DESCRIPTION OF THE INVENTION

Recombinantly-derived somatotropins without the additional Asp-Gln substitutions or with other substitutions are prepared in accordance with the present invention, as well.

Recombinant Porcine Somatotropin

```
H-Met-Asp-Gln-Phe-Pro-Ala-Met-Pro-Leu-Ser-Ser-
Leu-Phe-Ala-Asn-Ala-Val-Leu-Arg-Ala-Gln-His-Leu-
His-Gln-Leu-Ala-Ala-Asp-Thr-Tyr-Lys-Glu-Phe-Glu-
Arg-Ala-Tyr-Ile-Pro-Glu-Gly-Gln-Arg-Tyr-Ser-Ile-
                                          55
Gln-Asn-Ala-Gln-Ala-Ala-Phe-Cys-Phe-Ser-Glu-Thr-
Ile-Pro-Ala-Pro-Thr-Gly-Lys-Asp-Glu-Ala-Gln-Gln-
Arg-Ser-Asp-Val-Glu-Leu-Leu-Arg-Phe-Ser-Leu-Leu-
Leu-Ile-Gln-Ser-Trp-Leu-Gly-Pro-Val-Gln-Phe-Leu-
Ser-Arg-Val-Phe-Thr-Asn-Ser-Leu-Val-Phe-Gly-Thr-
Ser-Asp-Arg-Val-Tyr-Glu-Lys-Leu-Lys-Asp-Leu-Glu-
Glu-Gly-Ile-Gln-Ala-Leu-Met-Arg-Glu-Leu-Glu-Asp-
Gly-Ser-Pro-Arg-Ala-Gly-Gln-Ile-Leu-Lys-Gln-Thr-
Tyr-Asp-Lys-Phe-Asp-Thr-Asn-Leu-Arg-Ser-Asp-Asp-
                                               166
Ala-Leu-Leu-Lys-Asn-Tyr-Gly-Leu-Leu-Ser-Cys-Phe-
Lys-Lys-Asp-Leu-His-Lys-Ala-Glu-Thr-Tyr-Leu-Arg-
          183                                191
Val-Met-Lys-Cys-Arg-Arg-Phe-Val-Glu-Ser-Ser-Cys-
Ala-Phe-OH.
```

Recombinant Bovine Somatotropin

H-Met-Asp-Gln-Phe-Pro-Ala-Met-Ser-Leu-Ser-Gly-
Leu-Phe-Ala-Asn-Ala-Val-Leu-Arg-Ala-Gln-His-Leu-
His-Gln-Leu-Ala-Ala-Asp-Thr-Phe-Lys-Glu-Phe-Glu-
Arg-Thr-Tyr-Ile-Pro-Glu-Gly-Gln-Arg-Tyr-Ser-Ile-
55
Gln-Asn-Thr-Gln-Val-Ala-Phe-Cys-Phe-Ser-Glu-Thr-
Ile-Pro-Ala-Pro-Thr-Gly-Lys-Asn-Glu-Ala-Gln-Gln-
Lys-Ser-Asp-Leu-Glu-Leu-Leu-Arg-Ile-Ser-Leu-Leu-
Leu-Ile-Gln-Ser-Trp-Leu-Gly-Pro-Leu-Gln-Phe-Leu-
Ser-Arg-Val-Phe-Thr-Asn-Ser-Leu-Val-Phe-Gly-Thr-
Ser-Asp-Arg-Val-Tyr-Glu-Lys-Leu-Lys-Asp-Leu-Glu-
Glu-Gly-Ile-Leu-Ala-Leu-Met-Arg-Glu-Leu-Glu-Asp-

Gly-Thr-Pro-Arg-Ala-Gly-Gln-Ile-Leu-Lys-Gln-Thr-
Tyr-Asp-Lys-Phe-Asp-Thr-Asn-Met-Arg-Ser-Asp-Asp-
166
Ala-Leu-Leu-Lys-Asn-Tyr-Gly-Leu-Leu-Ser-Cys-Phe-
Arg-Lys-Asp-Leu-His-Lys-Thr-Glu-Thr-Tyr-Leu-Arg-
183 191
Val-Met-Lys-Cys-Arg-Arg-Phe-Gly-Glu-Ala-Ser-Cys-
Ala-Phe-OH.

Recombinant $Cys^{112, 122,} Glu^{183,191}$ porcine somatotropin

H-Met-Asp-Gln-Phe-Pro-Ala-Met-Pro-Leu-Ser-Ser-
Leu-Phe-Ala-Asn-Ala-Val-Leu-Arg-Ala-Gln-His-Leu-
His-Gln-Leu-Ala-Ala-Asp-Thr-Tyr-Lys-Glu-Phe-Glu-
Arg-Thr-Tyr-Ile-Pro-Glu-Gly-Gln-Arg-Tyr-Ser-Ile-
Gln-Asn-Ala-Gln-Ala-Ala-Phe-Cys-Phe-Ser-Glu-Thr-
Ile-Pro-Ala-Pro-Thr-Gly-Lys-Asp-Glu-Ala-Gln-Gln-
Arg-Ser-Asp-Val-Glu-Leu-Leu-Arg-Phe-Ser-Leu-Leu-
Leu-Ile-Gln-Ser-Trp-Leu-Gly-Pro-Val-Gln-Phe-Leu-
112
Ser-Arg-Val-Phe-Thr-Asn-Cys-Leu-Val-Phe-Gly-Thr-
122
Ser-Asp-Arg-Val-Cys-Glu-Lys-Leu-Lys-Asp-Leu-Glu-
Glu-Gly-Ile-Gln-Ala-Leu-Met-Arg-Glu-Leu-Glu-Asp-
Gly-Ser-Pro-Arg-Ala-Gly-Gln-Ile-Leu-Lys-Gln-Thr-
Tyr-Asp-Lys-Phe-Asp-Thr-Asn-Leu-Arg-Ser-Asp-Asp-
Ala-Leu-Leu-Lys-Asn-Tyr-Gly-Leu-Leu-Ser-Cys-Phe-
Arg-Lys-Asp-Leu-His-Lys-Thr-Glu-Thr-Tyr-Leu-Arg-
183 191
Val-Met-Lys-Glu-Arg-Arg-Phe-Val-Glu-Ser-Ser-Glu-
Ala-Phe-OH.

Recombinant Bovine Somatotropin

H-Met-Asp-Gln-Phe-Pro-Thr-Ile-Pro-Leu-Ser-Arg-
Leu-Phe-Asp-Asn-Ala-Met-Leu-Arg-Ala-His-Arg-Leu-
His-Gln-Leu-Ala-Phe-Asp-Thr-Tyr-Gln-Glu-Phe-Glu-

```
Glu-Ala-Tyr-Ile-Pro-Lys-Glu-Gln-Lys-Tyr-Ser-Phe-
                                56
Leu-Gln-Asn-Pro-Gln-Thr-Ser-Leu-Cys-Phe-Ser-Glu-
Ser-Ile-Pro-Thr-Pro-Ser-Asn-Arg-Glu-Glu-Thr-Gln-
Gln-Lys-Ser-Asn-Leu-Glu-Leu-Leu-Arg-Ile-Ser-Leu-
Leu-Leu-Ile-Gln-Ser-Trp-Leu-Glu-Pro-Val-Gln-Phe-
Leu-Arg-Ser-Val-Phe-Ala-Asn-Ser-Leu-Val-Tyr-Gly-
Ala-Ser-Asp-Ser-Asn-Val-Tyr-Asp-Leu-Leu-Lys-Asp-
Leu-Glu-Glu-Gly-Ile-Gln-Thr-Leu-Met-Gly-Arg-Leu-
Glu-Asp-Gly-Ser-Pro-Arg-Thr-Gly-Gln-Ile-Phe-Lys-
Gln-Thr-Tyr-Ser-Lys-Phe-Asp-Thr-Asn-Ser-His-Asn-
Asp-Asp-Ala-Leu-Leu-Lys-Asn-Tyr-Gly-Leu-Leu-Tyr-
167
Cys-Phe-Arg-Lys-Asp-Met-Asp-Lys-Val-Glu-Thr-Phe-
                        184
Leu-Arg-Ile-Val-Gln-Cys-Arg-Ser-Val-Glu-Gly-Ser-
191
Cys-Gly-Phe-OH.
```

**Derivatized somatotropins**

Derivatized somatotropins are prepared by dissolution or dispersion of protein in water or an aqueous solution of guanidine hydrochloride, sodium bicarbonate or the like, which is adjusted to pH 8.4 with an aqueous base, such as sodium or ammonium hydroxide. If reduction of a disulfide linkage is required, a dithiotheritol, i.e. (DL-threo-1, 4-dimercapto-2,3-butanediol DTT) slowly is then added to this solution. The addition is generally conducted under an atmosphere of nitrogen at room temperature. To the resulting solution is added the derivatizing agent. The mixture is stirred for about one to four hours. Progress of the reaction is monitored by Ellman titration of unmodified thiol groups or by gel electrophoresis. After the reaction is complete, it is desalted by ultrafiltration. The solution is concentrated and then subjected to several cycles of redilution with water, aqueous guanidine hydrochloride or the like, followed by ultrafiltration. The residue from the final filtration is then lyophilized to yield the derivatized protein.

The preparation of two of the derivatizing agents proceeds from N-Fmoc-Asp($^t$bu)OH and is depicted in Flow Chart 1 and Flow Chart 2. The amine derived from a polyethylene glycol monomethyl ether (PEG-OMe) is readily available using standard methodology. This amine is condensed with N-Fmoc-Asp($^t$bu)OH using dicyclohexylcarbodiimide (DCC) or other related dehydrating agent. Removal of the Fmoc protecting group utilizing standard protocols affords (I). Condensation of (I) with iodoacetic acid is effected with DCC followed by removal of the tert-butyl ester affording the alkylating agent. It is readily apparent that similar derivatives in which the polymer is attached to only the b-carboxyl or both the a and b carboxyl groups are prepared depending upon the starting material used.

Flow Chart 1

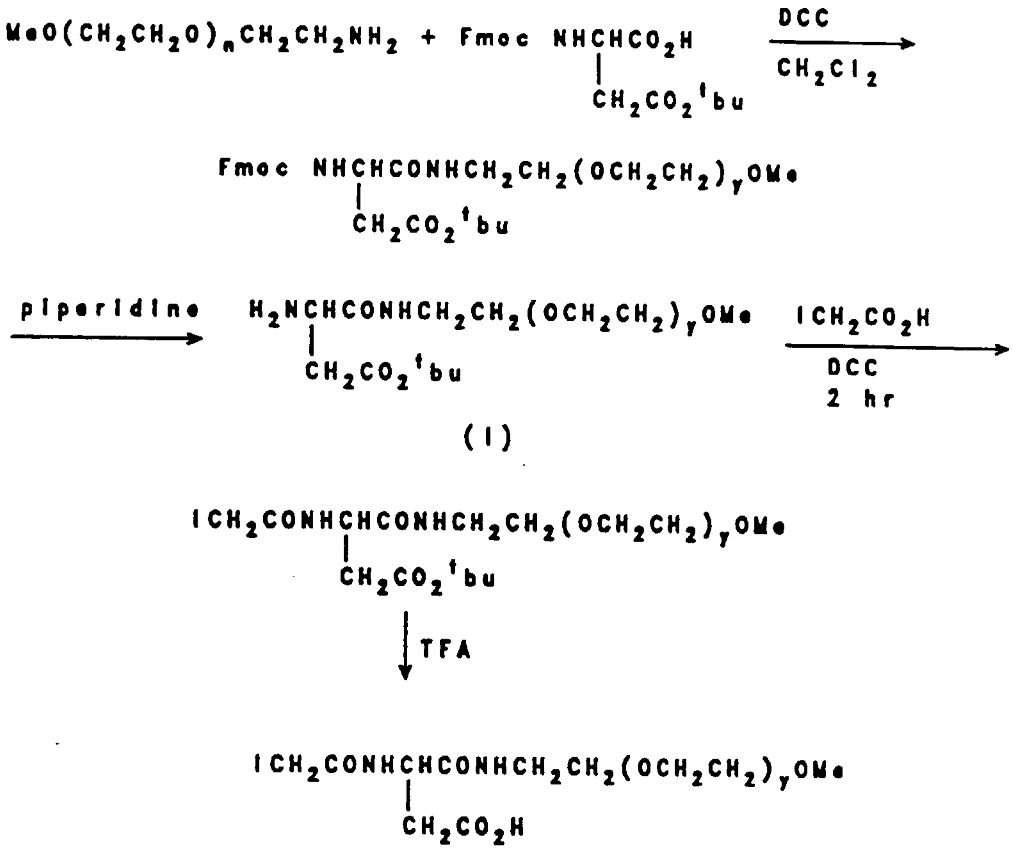


(I)

(II)

The succinic acid derivative (Flow Chart II) also is prepared from N-Fmoc-Asp($^t$bu)OH. The free acid is converted into the p-nitrobenzoate (PNB) ester with DCC and the amino group deprotected with piperidine.

Flow Chart 2

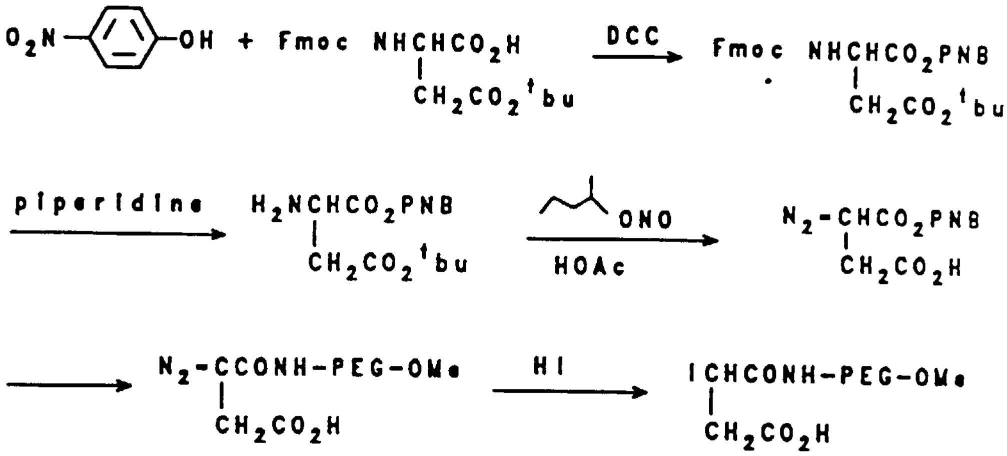


(III)

The amino group is converted to the diazonium salt (i-amyl nitrate/HOAc). Displacement of the active PNB ester with $H_2N$-PEG-OMe introduces the polymeric side chain. Treatment with HI results in simultaneous displacement of the diazo linkage with iodide and hydrolysis of the tert-butyl ester protecting group to afford the end result.

If N-protected 3-aminobutyric acid (Flow Chart 3) is utilized in place of Fmoc-N-Asp($^{+}$bu)OA, the corresponding monofunctionalized iodoacetamide derivative is prepared. Likewise, PEG-iodoacetates are directly prepared by DCC-mediated coupling.

It should be apparent that the linkage of the polymer can be through either an amide or ester. These two functional groups have different susceptability to hydrolysis and the preferred mode of attachment will vary with the specific application. Furthermore, the PEG polymers are available in a variety of molecular weight ranges all of which are potentially useful. Also, the nature of the polymer need not be limited to polyethylene glycol or polypropylene glycol. Other potentially useful polymers include, but are not limited to, polyvinyl alcohol, dextrans and carbohydrates, polypyrrolidone.

## Flow Chart 3

Fmoc $NH(CH_2)_3CO_2H + NH_2CH_2CH_2(OCH_2CH_2)_7OMe$

DCC

$CH_2Cl_2$

TFA

Fmoc $NH(CH_2)_3CONHCH_2CH_2(CH_2CH_2)_7OMe$

piperidine

$H_2N(CH_2)_3CONHCH_2CH_2(OCH_2CH_2)_7OMe$

$ICH_2CO_2H$

DCC

TFA

$ICH_2CONH(CH_2)_3CONHCH_2CH_2(OCH_2CH_2)_7OMe$

$MeO-(CH_2CH_2O)_7H + ICH_2CO_2H$

DCC

$CH_2Cl_2$

pyridine

$ICH_2CO_2CH_2CH_2(OCH_2CH_2)_7OMe$

**Site Directed Mutagenesis**

Preparation of a modified (substituted) recombinant animal (or human) somatotropins of this invention can be achieved by site directed mutagenesis. Currently-utilized techniques for the alteration of the DNA sequence of a cloned segment of DNA at a specific defined site require the production of a single stranded form of that DNA. The single stranded DNA is annealed to a synthetic oligonucleotide which is complementary to a portion of it, except that the oligonucleotide contains within it a region of mismatch. The region of mismatch is usually located in the central portion of the oligonucleotide. The annealed mixture is then made double stranded and covalently closed by the addition of E. coli DNA polymerase I, large fragment and deoxynucleotide triphosphates in the presence of T4 DNA ligase and adenosine 5' triphosphate. The double stranded DNA is then transformed into an appropriate E. coli strain where the mismatched region of the DNA is repaired and replicated. Two populations of clones are obtained. Dependant on which strand is chosen as the template for repair synthesis, a clone either contains the wild type or the altered (mutated) sequence. The clones which contain the mutated sequence, i.e. that which corresponds to the sequence of the oligonucleotide, are selected by hybridisation to the radioactively-labelled oligonucleotide. Due to the mismatch between the oligonucleotide and the wild type sequence, the radioactively-labelled oligonucleotide is more stably bound to the clones which contain the mutated sequence. Incubation at an appropriate temperature therefore differentiates between wild type and mutated clones. The alterations in the identified clones then are confirmed by DNA sequencing of the relevant regions.

One aim of mutagenesis is to generate a preferably-recombinantly-derived protein somatotropin (rpST) somatotropin molecule incapable of the formation of the small loop disulphide bond. The small loop disulphide is located between cysteines at 183 and 191. An additional aim is to incorporate two cysteine residues in the omega loop located at residues 102-112 which has been identified as a site of proteolytic cleavage. These newly incorporated cysteines are further modified using methods described herein. The numbering system is as described. The basic protocol used to obtain the required mutant is hereinafter described.

Substitution of the Ser102 and Tyr112 residues are achieved by using two oligonucleotides. The two oligonucleotides have the following sequence:

5' TTC ACC AAC TGT CTG GTG TTT 3' Ser102

5' GAC CGC GTC TGT GAG AAG CTG 3' Tyr112

The template single stranded DNA is pGEM3z(f+)pST-SX DNA. This single stranded DNA has already been modified to substitute two Glu residues for the cysteines normally found at positions 183 and 191 (This DNA is deposited in an expression vector PR0211, as indicated hereinbelow). 2000ng of single stranded PGEM3z(f+)pST-SX DNA is mixed with 50ng of AA1 oligonucleotide, which has previously been 5' phosphorylated with adenosine 5' triphosphate and polynucleotide kinase. The mixture is heated at 65°C for 7 minutes and then kept at room temperature for 10 minutes. This protocol anneals the oligonucleotide to the single stranded DNA. The annealed DNA is then converted to a double stranded covalently closed form by the addition of ATP, dNTP's (a mixture of the four deoxyribonucleotide 5' triphosphates), T4 DNA ligase and DNA polymerase I large fragment. The mixture is incubated for 1 hour at room temperature. The mixture is then transformed into HB101 competent cells by standard procedures. After overnight incubation at 37°C, colonies are transferred onto nitrocellulose filters and processed for hybridisation by standard protocols. Transformed colonies were identified using a radiolabelled oligonucleotide probe. Plasma DNA is prepared from these colonies, introduced into HB101 competent cell as described previously and rescreened with the radiolabelled oligonucleotide probe. Plasmid DNA is again prepared from colonies which hybridize with the probe. Plasmid DNA from one clone containing the S102C mutation, designated pGEM-3z(f+)pST-SXE-S102C is introduced into JM101 competent cells by transformation and single stranded DNA prepared from purified phage derived from a single transformant.

To mutate the tyrosine at position 112, a second oligonucleotide with following sequence is used:

5' GAC CGC GTC TGT GAG AAG CTG 3'

The process of mutagenesis is identical to that described above except that the template DNA is pGEM-3z(f+) pST-SXE-S102C (this is the clone isolated from the mutagenesis which has the serine at position 102 converted to cysteine) and glutamic acids substituted for cysteines at 183 and 191. The final wash temperature is 56°C. DNA sequencing reveals that the identified clones have the expected sequence. The mutated clone is designated pGEM-3z (f+)pST-SXE-S102C, Y112C.

The altered (mutant) clones are then reconstructed into the bacterial expression plasmid pR0211 (ATCC Accession Number 40483, deposited August 23, 1988) as described in Figure 5 of EPO publication 173,280. The pGEM clone is cut with EcoRI and HindIII and the pST gene fragment isolated. pRO211 is digested with the same enzymes treated with calf intestinal alkaline phosphatase and the large fragment isolated. The two pieces are ligated together with T4 DNA ligase and transformed into an appropriate bacterial strain, e.g. E. coli N99cI deposited.

In this strain a wild type g repressor is present. This prevents expression from the $P_L$ promoter in pRO211. Once the appropriate construction is isolated, it is then transferred into bacterial strains which contain a temperature sensitive g repressor, e.g. (ATCC Accession Number 67766, deposted August 23, 1988) E. coli 4200. In these strains, the expression of pST is dependent on temperature. At 42°C, the repressor is inactive and expression occurs. At this stage, pST is prepared by procedures contained in EP 173,280 wherein expression occurs (incorporated herein by reference thereto). Expression of pST is not limited to these particular E. coli strains. Other strains with appropriate properties are substituted. The plasmid expression vectors are deposited with the ATCC. The bacterial strains are deposited separately.

Other amino acid substitutions can be made by the above procedures utilizing the appropriate codons and oligonucleotides. Similarly, these procedures are employed to modify a variety of animal or human somatotropins.

**Method of Use**

Advantageously, the novel animal somatotropins, of the present invention are useful for alleviating disease states in animals; improving the growth rate of animals, especially meat producing animals; and increasing the efficiency of feed utilization thereby. Some of these compounds also are effective for enhancing the carcass quality of said animals, i.e. increasing the lean meat to fat ratio of said animals. Moreover, some of the compounds of the present invention are effective for increasing milk production in lactating animals and improving wool production in sheep and other animals raised for coats.

While the derivatized somatotropins of this invention are effective for treatment of animals to achieve the biological improvements described above,

the improved effectiveness and usefulness of the derivatized animal somatotropins of the invention can be attributed, in part, to the inhibition of aggregation of the somatotropin produced by the derivatization of said somatotropins. Such inhibition permits the preparation of markedly improved sustained release delivery systems which are not readily or effectively achieved with native somatotropins or recombinant somatotropins which are not derivatized as described by the present invention.

It is also found that the derivatized somatotropins of the present invention are highly stable and essentially free of aggregation due to dimer formation. Moreover, the derivatized somatotropins of the invention lend themselves to use in the preparation of significantly improved sustained release compositions.

Where native animal somatotropins have been found to aggregate in a single day, parenteral compositions containing the modified or derivatized somatotropins of this invention continue to release the modified or derivatized somatotropin for 10 days or more.

**Compositions**

In practice, the compositions of the present invention are generally administered to the animals by injection in the form of biologically active parenteral compositions. Among the parenteral compositions useful for administration of the recombinant animal proteins of this invention are gels, pastes, microspheres, microcapsules, implants and the like. As such, there is considerable interest in providing dosage forms of biologically active substances which release the substance in a controlled manner and thus, reduce the frequency of administration.

The development of sustained release compositions of biologically active macromolecules presents special problems due to their complex modes of action and intricate structures of the macromolecules. Thus, the development of effective sustained release compositions that contain the biologically active somatotropin is required.

The compositions useful for this type of administration are prepared by dissolving the modified or derivatized animal somatotropin in dilute ammonium hydroxide and then adding a solution of an alkali metal benzoate, laurate, carbonate or the like thereto. A nonionic surfactant is thereafter admixed with the solution and the resulting mixture spray dried. The thus formed solids are then admixed with molten fat or wax or a mixture thereof and the resulting molten mixture sprayed through an air/liquid spray nozzle equipped with a heated jacket to maintain the incoming air and the molten phase at a temperature above the melting point. The microspheres are formed as the molten droplets cool. These are collected on a series of sieves in the desired size range of about 45 to 180 microns and retained for use. Microspheres which are not of the desired size range are recycled. Alternatively, the homogeneous mixture is fed onto a centrifugal disc and the microspheres thus formed collected as above, or the molten mixture are cooled and milled to the desired average particle size range.

The biologically active microspheres are then dispersed in a pharmaceutically and pharmacologically acceptable liquid vehicle for injection into the animal. The microsphere-liquid composition is generally administered by subcutaneous injection under the skin of the animal usually in the vicinity of the head, neck or ears.

The derivatized animal somatotropins of the present invention also are prepared as biocompatible implants which are injected under the skin of the animal using a conventional pellet implant gun. The compositions are prepared by admixing a powdered derivatized somatotropin with a wax such as castor wax or with a mixture of a copoly(glycolide/lactide), magnesium hydroxide, and a condensate of ethylene oxide prepared with a hydrophobic base formed by condensation of propylene oxide with propylene glycol. The thus formed compositions are then introduced into a pelleting press and formed into cylindrical pellets about 1/8 inch in diameter. The thus formed pellets are administered with a conventional pellet implant gun.

The present invention is further illustrated by the examples set forth hereinbelow.

**EXAMPLE 1**

**Preparation of N-Fmoc-Asp($^t$bu)NH-PEG-OMe**

To solution of 1.24 g (3mM) of Fmoc Asp($^t$bu)-OH and 40 mL of $CH_2Cl_2$ is added 0.3 g (1.5 mM) of dicyclohexylcarbodiimide and the reaction mixture stirred at room temperature for one hour. The precipitated dicyclohexylurea is filtered off, and 3 g of polyethylene glycol (PEG) amine with an average molecular weight ($\overline{M}w$) of 2000 is added. The reaction is stirred overnight at room temperature. The methylene chloride is evaporated and the residue dissolved in 300 mL of $H_2O$. This solution was filtered, then ultrafiltered through a YM-2 Amicon membrane and lyophilized to afford 3 g of product.

**EXAMPLE 2**

**Preparation of $H_2N$-Asp($^t$bu)NH-PEG-OMe**

3 g of Fmoc-NHAsp($^t$bu)NH-PEG-OMe from Example 1 is added to 30 mL of piperidine-methylene chloride (1:1 v/v) at room temperature and stirred for 30 minutes. The methylene chloride is evaporated and the residue is dissolved in 200 mL of $H_2O$ and ultrafiltered through an Amicon YM-2 membrane. The remaining aqueous solution is lyophilized to yield 2.5 g of product.

**EXAMPLE 3**

**Preparation of $ICH_2$CONHAsp($^t$bu)NH-PEG-OME**

To a solution of 0.427 g of iodoacetic acid and 20 mL of methylene chloride is added 0.237 g of DCC and the reaction mixture is stirred at room temperature for one hour. The precipitated dicyclohexylurea is filtered and 2.5 g of $H_2N$-Asp($^t$bu)NH-PEG-OMe is added and the mixture stirred for 3 hours at room temperature (until ninhydrin test is negative). The methylene chloride was evaporated and the residue dissolved in water and ultrafiltered through an Amicon YM-2 membrane. The solution is then lyophilized to yield 2.6 g of product.

**EXAMPLE 4**

**Preparation of $ICH_2$CONHAsp(OH)NH-PEG-OMe**

2.6 g of $ICH_2$CONH-Asp($^t$bu)NH-PEG-OMe is dissolved in 40 mL of trifluoroacetic acid/methylene chloride (1:1 v/v) and the mixture is stirred at room temperature for one hour. The solvents are evaporated and the residue dissolved in about 200 mL of $H_2O$. The solution is ultrafiltered through an Amicon YM-2 membrane and neutralized at 0° with ammonium hydroxide. Lyophilization affords 2 g of $ICH_2$CONHAsp(OH)NH-PEG-OMe.

**EXAMPLE 5**

**Preparation of [(MeO-PEG-NHCOAsp (OH)NHCOCH$_2$)Cys $^{183,191}$]-rpST**

To a solution of 400 mg of r-pST in 200 mL of 0.5 M $NH_4HCO_3$ (pH=8.4) is added 28.0 mg of dithiothreitol and the reaction mixture stirred for 1 hour. To this reduced r-pST is added 1 g of MeO-PEG-NHCO-Asp(OH)NHCOCH$_2$I and the mixture allowed to stir for 3 hours. Ellman's test indicates free thiols remain, and so an additional one g of the iodoacetamide derivative was added and the reaction mixture stirred at room temperature for 18 hours. The reaction mixture is diluted, ultrafiltered through an Amicon YM-10 membrane and lyophilized to yield 400 mg of product.

**EXAMPLE 6**

**Preparation of Fmoc NH Asp($^t$bu)COPNB**

To a solution of 8 g Fmoc-Asp($^t$bu)OH and 250 mL of methylene chloride is added 2.01 gm of DCC and the resulting solution stirred at room temperature for 1.5 hours. The precipitated dicyclohexylurea was filtered and 1.35 g 4-nitrophenol is added and the reaction mixture stirred for 18 hours. The solution is cooled and washed with cold 5% $Na_2CO_3$ and brine. After drying, the solution is evaporated to yield 4.27 g of product.

**EXAMPLE 7**

**Preparation of $H_2NCH(CO_2PNB)CH_2CO_2{}^t$bu**

2 g of Fmoc NHCH($CO_2$PNB)$CH_2CO_2{}^t$bu was added to a 30 mL of an ice cold mixture of piperidine and methylene chloride (1:1 v/v) and stirred for 2 hours at 0°C. The solvents were evaporated and the residue redissolved in methylene chloride and washed with cold 0.1 N HCl, water and brine. The resulting solution was dried and evaporated to yield 0.83 g of product.

**EXAMPLE 8**

**Preparation of $N_2CH(CONH\text{-}PEG\text{-}OMe)CH_2CO_2{}^tbu$**

A solution of 2.0 g of $H_2NCH(CO_2PNB)CHCO_2\text{-}{}^tbu$, 0.91 g i-amyl nitrite, 3 drops of HOAc and 100 mL of $CH_2Cl$ were refluxed for 2 hours. The volatile solvents were evaporated and the residue redissolved in 50 mL of $CH_2Cl_2$ and 5 g of MeO-PEG-$NH_2$ were added. The mixture is stirred evernight at room temperature. The solvents were evaporated and the residue dissolved in $H_2O$ and ultrafiltered through an Amicon YM-2 membrane. Lyophilization yielded 1.48 gm of product.

**EXAMPLE 9**

**Preparation of $ICH(CONH\text{-}PEG\text{-}OMe)CH_2CO_2H)$**

A solution of 0.5 g $N_2C(CONH\text{-}PEG\text{-}OMe)CH_2CO_2H$ is dissolved in 50 mL of $CHCl_3$ and cooled to 0°C. 2 mL of HI is added carefully and the reaction stirred for 30 minutes at 0°C and then allowed to warm to 28°C for 2 hours. The solution is evaporated, dissolved in 30 mL $H_2O$ and ultrafiltered through an Amicon YM-2 membrane. After lyophilization 0.3 g of product remains.

**EXAMPLE 10**

**Preparation of $[(MeO\text{-}PEG\text{-}NHCOCH(CH_2CO_2H)Cys^{183,191})]$**

To a solution of 500 mg of rpST in 250 mL of 0.5 N $NaHCO_3$ (pH=8.4) is added 35 mg of dithiothreitol and the reaction mixture stirred for 1 hour. To the reduced rpST is added 2.0 g of MeO-PEG-NHCOCHI($CH_2$-$CO_2H$) and the reaction mixture stirred overnight at room temperature. The reaction mixture is diluted with $H_2O$ and ultrafiltered through an Amicon YM-10 membrane and lyophilized to yield 510 mg of product.

**EXAMPLE 11**

**Preparation of MeO-PEG-$OCOCH_2I$**

To a solution of 5 g of iodoacetic acid in 250 mL of methylene is added 2.76 g of DCC. The reaction mixture is stirred at ambient temperature for 2 hours and the precipitated dicyclohexylurea removed by filtration. To the remaining solution is added 20 g of MeO-PEG-OH and the reaction mixture stirred overnight at room temperature. The solution is then cooled and transferred to a separation funnel and extracted with 15 x 10 mL of cold saturated $NaHCO_3$. The remaining methylenechloride solution is evaporated and the residue dissolved in 30 mL of $H_2O$. This solution was ultrafiltered through an Amicon YM-2 membrane and finally lyophilized to yield 5.4 g of product.

**EXAMPLE 12**

**Introduction of cysteines into the omega loop of porcine somatotropin using two synthetic oligonucleotides**

A synthetic oligonucleotide designated S102C has the following sequence:

5' TTC ACC AAC TGT CTG GTG TTT 3'

This oligonucleotide differs from the analogous DNA sequence of the rpST gene in that the DNA encoding the serine codon at position 102 is replaced with DNA encoding cysteine. Therefore, the serine present at position 102 is replaced with cysteine.

Single stranded pGEM-3z(f+)pST-SX DNA is the substrate for mutagenesis. This DNA is composed of a modified rpST gene contained in the commercially available phagemid, pGEM-3z(f+). The modifications of the rpST gene result in the alteration of the DNA sequence to allow for the introduction of a Sac I restriction endonuclease cleavage site at positions 225-230 of the coding region and for the introduction of an Xba I restriction endonuclease cleavage site at positions 285-290. These alterations in the DNA sequence do not change the amino acid sequence of the rpST protein. Two other modifications of the rpST gene result in the alteration of the DNA sequence to allow for the replacement of

the cysteine residues at positions 183 and 191 each with glutamic acid. An EcoRI/HindIII fragment containing this modified rpST-SXE gene is cloned into pGEM-3z(f+) DNA cleaved with these restriction endonucleases using standard procedures, resulting in phagemid pGEM-3z(F+)pST-SX. The single stranded pGEM-3z(F+) DNA is prepared from purified phage by standard protocols. 2000ng of this DNA is mixed with 100 ng of the C183del oligonucleotide, the latter of which has been phosphorylated at its 5' end previously with adenosine 5' triphosphate and polynucleotide kinase. The mixture is contained in a total volume of 10 ml in 1X annealing buffer (1X annealing buffer is 75 mM KCl and 5 mM Tris-Cl, pH 8.0). The mixture is heated at 65°C for 7 minutes followed by a 10 minute incubation at room temperature. This procedure permits the oligonucleotide to anneal to the single stranded substrate DNA. Annealed molecules are extended and converted to covalently closed, double stranded DNA by the addition of 22 ml $H_2O$, 1ml 20 mM ATP, 2 units each of T4 DNA ligase, and DNA polymerase I large fragment (for unit definition, see New England Biolabs catalogue, 1986), 2 ml 20 X dNTP's (a mixture of the four deoxyribonucleotide 5' triphosphates each at a concentration of 2 mM) and 4 ml 10X fill in buffer (1X fill in buffer is 27.5 mM Tris-Cl pH 7.5, 15 mM MgCl2, 2mM DTT). After a one hour incubation at room temperature, half of the reaction is introduced into HB101 competent cells by a standard transformation protocol. After overnight incubation at 37°C, colonies are transferred onto nitrocellulose filters and processed for hybridization by standard procedures. Oligonucleotide S102C is used for detection of the desired mutation by radiolabelling its 5' end with q-32P-ATP and polynucleotide kinase. After a three hour prehybridization at 37°C in 5X SSC, 1X Denhardt's and 150 mg/ml yeast tRNA, the radiolabelled oligonucleotide is added and allowed to hybridize with the DNA on the filters overnight at 37°C. The filters are washed for 30 minutes at 37°C in 5X SSC, followed by a 30 minute wash in TAC at 58.0°C. During this latter wash, only those colonies whose plasmid DNA contains the desired mutation will continue to hybridize with the radiolabelled oligonucleotide probe.
These colonies are detected after exposure of the washed filters to X-ray film. Plasmid DNA is prepared from several of these colonies from the original petri plate, introduced into HB101 competent cells as described previously, and rescreened with the radiolabelled oligonucleotide probe as above. Plasmid DNA is prepared from colonies whose DNA hybridizes with the probe and analyzed for the desired DNA sequence. Plasmid DNA from one clone containing the S102C mutation, designated pGEM-3z(f+)pST-SXE-S102C, is introduced into JM101 competent cells by transformation and single stranded DNA prepared from purified phage derived from a single transformant.

To replace the tyrosine-encoding DNA at position 112 with cysteine-encoding DNA, the following oligonucleotide, designated Y112C, is synthesized:

**5' GAC CGC GTC TGT GAG AAG CTG 3'**

This oligonucleotide differs from the analogous DNA in the rpST gene in that the DNA encoding the tyrosine codon at position 112 is replaced by DNA encoding cysteine.

The template single stranded DNA from pGEN-3z(f+)pST-SXE-S102C and Y112C oligonucleotide are annealed, extended and ligated as before. The mixture is introduced into HB101 competent cells and transformants are analyzed for the presence of the mutation exactly as described for S102C. The Y112C oligonucleotide is used as the radiolabelled detection probe. Hybridization and washing conditions are exactly as described for S102C. Several clones containing the Y112C mutation are identified from DNA sequence analysis. Plasmid DNA containing the S102C and Y112C mutations is designated pGEM-3z(f+)pST-SXE-S102C, Y112C.

## EXAMPLE 13

### Reconstruction into appropriate bacterial expression plasmids

The altered (mutant) clones are reconstructed into the bacterial expression plasmid pRO211 (described in EP 173280), incorporated herein by reference thereto. The M13 clones are cut with ECoRI and HindIII and the pST gene fragment isolated. pRO211 is cloned with the same enzymes treated with calf intestinal alkaline phosphatase and the large fragment isolated. The two pieces are ligated together with T4 DNA ligase and transformed into an appropriate bacterial strain, e.g. N99cI$^+$. In this strain a wild type g repressor is present. This prevents expression from the $P_2$ promoter in pRO211. Once the appropriate construction has been isolated, it is then transferred into bacterial strains which contain a temperature sensitive g repressor, e.g. 4200. In these strains, the expression of rpST is dependent on temperature. At 42°C, the repressor is inactive and expression occurs. At this stage, rpST can be prepared by procedures contained in EP 173280, incorporated herein by reference thereto. The E. coli strain which are used for expression of the pST gene product are not limited to E. coli 4200. Any appropriate E. coli strain can be utilized.

Following the procedures of Examples 1, 2 and 3 above, but substituting the appropriate codons for alanine, serine, glutamic acid, arginine, trypophan, or asparagine for cysteine at positions 183 and 191 converts TGT for cysteine to

TCT, AGC or AGT for serine, GAG, or GAA for glutamic acid, CGN, AGA or AGG for arginine, AAT or AAC for asparagine, GCG for alanine, or TGG for tryptophan at said position.

**EXAMPLE 14**

**Preparation of [(MeO-PEG-OCOCH$_2$)Cys$^{102,112}$,Glu$^{183,191}$]-rpST**

To a solution of 200 mg of Cys$^{102,112}$ Glu-$^{183,191}$ rpST in 150 mL of 0.5N $NaH_2CO_3$ (pH=8.4) is added 28 mg of dithothreital and the solution stirred for 1 hour. To the solution was added 500 mg of MeO-PEG-$O_2CCH_2I$ ($\overline{M}$w-550) and the reaction mixture stirred for 8 hours at room temperature. The reaction mixture is ultrafiltered through an Amicon YM-10 membrane to yield 135 mg of product.

**EXAMPLE 15**

**Preparation of [(Meo-PEG-NHCOCH$_2$)Cys$^{127}$ ]Interleukin-2**

To a solution of 25 mg of Interleukin-2 dissolved in 25 mL of 0.5N $NaH_2CO_3$ (pH-8.4) is added 20 mg of MeO-PEG-NHCOCH$_2$I ($\overline{M}$w-2000) and the reaction mixture stirred for 48 hours until no free thiol is detected. The reaction mixture is ultrafiltered through an Amicon YM-10 membrance to yield 16 mg of product.

**EXAMPLE 16**

**Preparation of [(MeO-PEG-OCOCH$_2$)Cys $^{183,191}$]r-pST**

To a solution of 400 mg of rpST in 250 mL of 0.5 N $NaHCO_3$ (pH=8.4) is added 28 mg of dithiothreitol and the solution stirred for 1 hour. To the solution is added 1.0 g of MeO-PEG-$O_2CCH_2I$ ($\overline{M}$w=2000) and the reaction mixture stirred for 6 hours at room temperature. The reaction mixture is ultrafiltered through an Amicon YM-10 membrane and finally lyophilized to yield 420 mg of product.

**EXAMPLE 17**

**Hypox rat test method for determining the growth enhancement of animals receiving derivatized recombinant animal somatotropin**

The efficacy of various derivatized recombinant animal somatotropins of the present invention in altering the growth rate of animals is determined utilizing the standard hypophysectomized (hypox) rat assay. The hypophysectomized rat does not produce its own growth hormone and is sensitive to injected bovine growth hormone. The response measured is growth over a period of time, such as 10 days.

Each of the hypox albino rats (Taconic Farms, Sprague Dawley-derived) is injected with a sufficient quantity of the compositions prepared in accordance with the Examples herein to provide a 10-day dose of 800 micrograms (80 micrograms/day) rPST in 0.2 ml of the formulation listed. Alternatively, each of the hypox albino rats is injected daily with 80 micrograms of rPST derivative.

**Test Procedures**

Prior to the test, the animals are weighed and the animals to be used for the test are selected based on body weight. only those animals whose body weights are one standard deviation from the mean body weight of the group are selected. The resulting group is then randomly divided into treatment groups consisting of eight rats/group by a computer generated randomization procedure. The test animals are then transferred to a clean cage and housed four rats/cage. On the initial day of the study the test animals are weighed and any animals with excessive weight gain or loss (±5 grams) are replaced. The animals are then assigned to test groups and treated.

At the end of the ten-day test period, total weight gain for each animal is recorded and the average weight gain per rat for each treatment determined. The results of these experiments, which are summarized in Table I below, demonstrate the bioactivity of these derivatives.

TABLE I

| Hypox Rat Test | | | | | | |
|---|---|---|---|---|---|---|
| Hypox Rat Data | | | | | | |
| | gms (growth) | | | | | |
| derivative | PEG $\bar{M}W$ | 0-2 days | 2-4 days | 4-7 days | 7-10 days | total |
| [[(Meo-PEG-$O_2$CCH-($CH_2$ $CO_2Na^+$) NHCO-$CH_2$]Cys183,191]-r-pST | 2000 | 8.6 | 6.3 | 9.0 | 7.5 | 31.4 |
| [(Meo-PEG-$O_2$C-$CH_2$)Cys183,191]-r-pST | 5000 | 8.8 | 5.0 | 10.3 | 7.3 | 31.3 |
| [(Meo-PEG-$O_2$C-$CH_2$)Cys183,191]-r-pST | 2000 | 9.8 | 4.8 | 8.9 | 5.9 | 29.3 |
| r-pST | - | 6.9 | 5.3 | 6.8 | 9.0 | 28.0 |

From the above data it is seen that each of the derivatized recombinant porcine somatotropins evaluated is biologically active and provides excellent growth of animals.

**EXAMPLE 18**

**Stability profiles of modified or derivatized recombinant animal somatotropins**

A concentrated solution of the recombinant animal somatotropin derivative (up to 100 mg/ml) in phosphate buffered saline pH 7.4 ($NaH_2PO_4$ $H_2O$ 3.45 gm, $Na_2HPO_4$ 3.55 gm, NaCl 9.50 gm dissolved in distilled water to 1000 ml) is prepared. This is filtered through a millipore sterile Millex-0.22um filter unit and 0.1 ml aliquots placed into tubes. These are placed in a 45°C oven and removed at the required intervals. The contents are then diluted with phosphate buffered saline. The supernatent is assayed for monomer and dimer content by HPLC. A mass balance is done; any precipitated material is recorded. Results are compared with the initial concentrations, and a stability profile documented.

Alternately, a somatotropin derivative exhibiting poor solubility at pH 7.4 is dissolved at a less preferred pH (4-10) or is evaluated as a suspension (see Table II).

TABLE II

| Sample | 7 days | 14 days | 21 days |
|---|---|---|---|
| | Fraction Soln. (Dimer) | Fraction Soln. (Dimer) | Fraction Soln.(Dimer) |
| [(MeO-PEG-$OCO_2CH_2$)$Cys^{183,191}$] r-pST | | | |
| $\bar{M}W$=2000 | 52 | - | - |
| $\bar{M}W$=5000 | - | 19 | - |
| [(MeO-PEG-NHCO)($CO_2^-Na^+$)CHNHCO$CH_2$]$Cys^{183,191}$ r-pST | | | |
| $\bar{M}W$=200 | | | 83 |
| r-pST technical | 85.9(29.4) | 65.4(45.5) | 59.8(51.7) |
| | 89.5(31.0) | 64.0(44.9) | 57.9(49.2) |

**EXAMPLE 19**

**Preparation of implants using [(MeO-PEG-$O_2$CC$H_2$)Cys-$^{183,191}$)rpST and evaluation of said implants by in vitro dissolution**

A set of implants are prepared from 96 mg of -270 mesh castorwax and 64 mg of powdered [(MeO-PEG-$O_2$CC$H_2$) $Cys^{183,191}$]rpST and rpST. The average molecular weight of the PEG is 2,000. The mixture is agitated, poured over a large surface petri dish and the $CH_2Cl_2$ allowed to evaporate at room temperature and then dried by vacuum drying. The dried residue is collected and formed in 1/8" diameter cylindrical implants using a Carver Press at about 1000 psig. The thus-formed implants weigh 60 to 70 mg each. The thus-prepared implants are then subjected to an in vitro

dissolution evaluation. Each of the implants is placed in a separate plastic tube containing 10 ml of a phosphate buffer solution (pH 7.4 with 0.05% Na azide) and the tubes placed in a shaking water rack where the tubes are shaken while the temperature of the water in the unit is maintained at 39°C. The tubes are shaken for one day, then the solutions removed from each tube and analyzed for rpST by HPLC and the solution discarded. New phosphate buffer solution is added to each tube and the tubes shaken for three additional days thereafter. The solutions from each tube is again analyzed for rpST by HPLC and (the solution again is discarded). New phosphate buffer are again added to each tube and the tube again shaken for three days then analyzed again for rpST.

The phosphate buffer solution is ($NaH_2$-$PO_4 \cdot H_2O$ 3.45 g, $Na_2HPO_4$ 3.55 g, Nacl 9.5 g dissolved in distilled water to 1000 ml (see Table III).

TABLE III

| Implant % Release | | | | |
|---|---|---|---|---|
| Implants pressed from a mixture of 60% Castorwax and 40% r-pST and coated with silicone tubing | | | | |
| | PEG | % Released | | |
| Compound | Mw | Day 1 | Day 4 | day 7 |
| r-pST | | 8.1 | 11.3 | 12.9 |
| [(MeO-PEG-$OCCH_2$)-$Cys^{183,191}$]pST | 2000 | 12.4 | 17.1 | 17.6 |

## Claims

### Claims for the following Contracting States : AT, BE, CH, LI, DE, DK, FR, GB, IT, LU, NL, SE

1. A derivatized somatotropin wherein at least one cysteine residue of said somatotropin is derivatized with substituents, said substituents comprising: ($CH_2CO_2R_1$), ($CH_2CONHR_1$), [CH($COR_2$ )($CH_2$)$_x$ ($COR_3$)], [$CH_2$CONHCH ($COR_2$)-($CH_2$ )$_x$ ($COR_3$)] or [$CH_2$CONH-($CH_2$)$_x$$COR_4$]; wherein $R_1$ is $CH_2CH_2(OCH_2CH_2)_y$-OMe; $R_2$ and $R_3$ are H or $R_4$;$R_4$ is $NHCH_2CH_2(OCH_2CH_2)_y$ OMe or $OCH_2CH_2(OCH_2$-$CH_2)_y$OMe wherein x is an integer of from 1 to 3 and y is an integer of from 10 to 300; with the proviso that $R_2$ and $R_3$ cannot simultaneously be H; provided that when the derivatized cysteine residue(s) of said somatotropin form a disulfide linkage prior to derivatization, both cysteines will bear the same sulfhydryl derivative.

2. A somatotropin according to Claim 1, wherein said somatotropin contains at least one cysteine residue in its natural form.

3. A somatotropin according to Claim 1, wherein said somatotropin has had a cysteine residue added or substituted in the somatotropin natural form.

4. A pharmaceutical composition comprising: a pharmacologically effective amount of a modified or derivatized somatotropin according to any of claims 1-3 or pharmaceutically acceptable salts thereof; and a pharmaceutically acceptable solid or liquid carrier therefore, wherein said composition is effective in increasing the growth rate of an animal over an extended period of time.

5. A composition according to Claim 9, wherein said composition is parenterally administered to said animal.

### Claims for the following Contracting States : ES, GR

1. A method of preparing a derivatized somatotropin wherein at least one cysteine residue of said somatotropin is derivatized with substituents, said substituents comprising: ($CH_2CO_2R_1$), ($CH_2CONHR_1$), [CH($COR_2$) ($CH_2$)$_x$ ($COR_3$)], [$CH_2$CONHCH($COR_2$)-($CH_2$)$_x$($COR_3$)] or [$CH_2$CONH-($CH_2$)X$COR_4$]; wherein $R_1$ is $CH_2CH_2(OCH_2CH_2)_y$-OMe; $R_2$ and $R_3$ are H or $R_4$; $R_4$ is $R_4$=$NHCH_2CH_2(OCH_2CH_2)_y$OMe or $OCH_2CH_2(OCH_2$-$CH_2)_y$ OMe wherein x is an integer of from 1 to 3 and y is an integer of from 10 to 300; with the proviso that $R_2$ and $R_3$ cannot simultaneously be H; provided that when the derivatized cysteine residue(s) of said somatotropin form a disulfide linkage prior to derivatization, both cysteines will bear the same sulfhydryl derivative, in which a derivatizing agent bearing substituting residues selected from ($CH_2CO_2R_1$), ($CH_2CONHR_1$), [CH($COR_2$) ($CH_2$)$_x$($COR_3$)], [$CH_2$-CONHCH($COR_2$)

$(CH_2)_x(COR_3)$], or [$CH_2CONH(CH_2)_xCOR_4$]; wherein $R_1$ is $CH_2CH_2$- $(OCH_2\ CH_2)_y$ OMe; $R_2$ and $R_3$ are H or $R_4$ ; $R_4$ is $NHCH_2\ CH_2\ (OCH_2CH_2)_y$ OMe or $OCH_2CH_2(OCH_2CH_2)_yOMe$; X is an integer of from 1 to 3 and Y is an integer of from 10 to 300; with the proviso that $R_2$ and $R_3$ cannot simultaneously be H; is reacted with said somatotropin; provided that if the derivatized cystein residue(s) of said somatotropin form a disulfide linkage prior to derivatization, said disulfide linkage is cleaved and reduced prior to derivatization.

**2.** The method according to Claim 1, wherein said somatotropin contains at least one cysteine residue in its natural form.

**3.** The method according to Claim 1, wherein said somatotropin has had a cysteine residue added or substituted in the somatotropin natural form.

**4.** A method for preparing a pharmaceutical composition in which a pharmacologically effective amount of a modified or derivatized somatotropin according to any of claims 1-3 or pharmaceutically acceptable salts thereof; and a pharmaceutically acceptable solid or liquid carrier therefore are formulated, wherein said composition is effective in increasing the growth rate of an animal over an extended period of time.

**5.** The method according to Claim 4, wherein said composition is parenterally administered to said animal.

**Patentansprüche**

**Patentansprüche für folgende Vertragsstaaten : AT, BE, CH, LI, DE, DK, FR, GB, IT, LU, NL, SE**

**1.** Derivatisiertes Somatotropin, in dem mindestens ein Cystein-Rest des Somatotropins mit Substituenten derivatisiert ist, wobei die Substituenten umfassen: $(CH_2CO_2R_1)$, $(CH_2CONHR_1)$, [$CH(COR_2)\ (CH_2)_x(COR_3)$], [$CH_2CONHCH(COR_2)$-$(CH_2)_x(COR_3)$] oder [$CH_2CONH$-$(CH_2)_xCOR_4$]; worin $R_1$ $CH_2CH_2(OCH_2CH_2)_y$-OMe ist; $R_2$ und $R_3$ H oder $R_4$ sind; $R_4$ $NHCH_2CH_2(OCH_2CH_2)_yOMe$ oder $OCH_2CH_2(OCH_2$-$CH_2)_yOMe$ ist, worin x eine ganze Zahl von 1 bis 3 ist und y eine ganze Zahl von 10 bis 300 ist; mit der Maßgabe, daß $R_2$ und $R_3$ nicht gleichzeitig H sein können; vorausgesetzt, daß, wenn der bzw. die derivatisierte(n) Cystein-Rest(e) des Somatotropins vor der Derivatisierung eine Disulfid-Verknüpfung bilden, beide Cysteine das gleiche Sulfhydryl-Derivat tragen.

**2.** Somatotropin nach Anspruch 1, in dem das Somatotropin mindestens einen Cystein-Rest in seiner natürlichen Form enthält.

**3.** Somatotropin nach Anspruch 1, in dem das Somatotropin einen Cystein-Rest aufweist, der zu der natürlichen Somatotropin-Form hinzugefügt oder in dieser substituiert worden ist.

**4.** Pharmazeutische Zusammensetzung, umfassend: eine pharmakologisch wirksame Menge eines modifizierten oder derivatisierten Somatotropins nach irgendeinem der Ansprüche 1 - 3 oder pharmazeutisch annehmbare Salze desselben; und einen pharmazeutisch annehmbaren festen oder flüssigen Träger für dasselbe, wobei die Zusammensetzung bei der Steigerung der Wachstumsgeschwindigkeit eines Tieres über eine ausgedehnte Zeitspanne wirksam ist.

**5.** Zusammensetzung nach Anspruch 4, bei der die Zusammensetzung dem Tier parenteral verabreicht wird.

**Patentansprüche für folgende Vertragsstaaten : ES, GR**

**1.** Verfahren zur Herstellung eines derivatisierten Somatotropins, in dem mindestens ein Cystein-Rest des Somatotropins mit Substituenten derivatisiert ist, wobei die Substituenten umfassen:
$(CH_2CO_2R_1)$, $(CH_2CONHR_1)$, [$CH(COR_2)\ (CH_2)_x(COR_3)$ ], [$CH_2CONHCH(COR_2)$-$(CH_2)_x(COR_3)$] oder [$CH_2CONH$-$(CH_2)_xCOR_4$]; worin $R_1$ $CH_2CH_2(OCH_2CH_2)_y$-OMe ist; $R_2$ und $R_3$ H oder $R_4$ sind; $R_4$ $NHCH_2CH_2$ $(OCH_2CH_2)_yOMe$ oder $OCH_2CH_2(OCH_2$-$CH_2)_yOMe$ ist, worin x eine ganze Zahl von 1 bis 3 ist und y eine ganze Zahl von 10 bis 300 ist; mit der Maßgabe, daß $R_2$ und $R_3$ nicht gleichzeitig H sein können; vorausgesetzt, daß, wenn der bzw. die derivatisierte(n) Cystein-Rest(e) des Somatotropins vor der Derivatisierung eine Disulfid-Verknüpfung bilden, beide Cysteine das gleiche Sulfhydryl-Derivat tragen, in welchem ein Derivatisierungsmittel, das

substituierende Reste trägt, die aus ($CH_2CO_2R_1$), ($CH_2CONHR_1$), [$CH(COR_2)(CH_2)_x(COR_3)$], [$CH_2CONHCH(COR_2)(CH_2)_x(COR_3)$] oder [$CH_2$-$CONH(CH_2)_xCOR_4$] ausgewählt sind; worin $R_1$ $CH_2CH_2$-$(OCH_2CH_2)_yOMe$ ist; $R_2$ und $R_3$ H oder $R_4$ sind; $R_4$ $NHCH_2CH_2(OCH_2CH_2)_yOMe$ oder $OCH_2CH_2(OCH_2CH_2)_yOMe$ ist; x eine ganze Zahl von 1 bis 3 ist und y eine ganze Zahl von 10 bis 300 ist; mit der Maßgabe, daß $R_2$ und $R_3$ nicht gleichzeitig H sein können; mit dem Somatotropin umgesetzt wird; vorausgesetzt, daß, wenn der bzw. die derivatisierte(n) Cystein-Rest(e) des Somatotropins vor der Derivatisierung eine Disulfid-Verknüpfung bilden, diese Disulfid-Verknüpfung vor der Derivatisierung gespalten und reduziert wird.

2. Verfahren nach Anspruch 1, in dem das Somatotropin mindestens einen Cystein-Rest in seiner natürlichen Form enthält.

3. Verfahren nach Anspruch 1, in dem das Somatotropin einen Cystein-Rest aufweist, der zu der natürlichen Somatotropin-Form hinzugefügt oder in derselben substituiert worden ist.

4. Verfahren zur Herstellung einer pharmazeutischen Zusammensetzung, in dem eine pharmakologisch wirksame Menge eines modifizierten oder derivatisierten Somatotropins nach irgendeinem der Ansprüche 1 - 3 oder pharmazeutisch annehmbare Salze desselben; und ein pharmazeutisch annehmbarer fester oder flüssiger Träger für dasselbe formuliert werden, wobei diese Zusammensetzung bei der Steigerung der Wachstumsgeschwindigkeit eines Tieres über eine ausgedehnte Zeitspanne wirksam ist.

5. Verfahren nach Anspruch 4, in dem die Zusammensetzung dem Tier parenteral verabreicht wird.

## Revendications

### Revendications pour les Etats contractants suivants : AT, BE, CH, LI, DE, DK, FR, GB, IT, LU, NL, SE

1. Dérivé de somatotropine dans lequel au moins un résidu de cystéine de ladite somatotropine est modifié avec des substituants, lesdits substituants comprenant: ($CH_2CO_2R_1$), ($CH_2CONHR_1$), [$CH(COR_2)$ $(CH_2)_x(COR_3)$], [$CH_2CONHCH$ $(COR_2)$- $(CH_2)_x(COR_3)$] ou [$CH_2CONH$-$(CH_2)_x$-$COR_4$]; dans lesquels $R_1$ est $CH_2CH_2(OCH_2CH_2)_yOMe$; $R_2$ et $R_3$ sont H ou $R_4$; $R_4$ est $NHCH_2CH_2(OCH_2CH_2)_yOMe$ ou $OCH_2CH_2(OCH_2CH_2)_yOMe$; où X est un entier compris entre 1 et 3 et Y est un entier compris entre 10 et 300; à condition que $R_2$ et $R_3$ ne puissent pas être simultanément H; pourvu que, lorsque le(s) dérivé(s) de la cystéine modifiée de ladite somatotropine forme(nt) une liaison disulfure avant la modification, les deux cystéines portent le même dérivé sulfhydryle.

2. Somatotropine selon la revendication 1, dans laquelle ladite somatotropine contient au moins un résidu de cystéine sous sa forme naturelle.

3. Somatotropine selon la revendication 1, dans laquelle ladite somatotropine a subi une addition ou une substitution d'un résidu de cystéine, sur la forme naturelle de la somatotropine.

4. Composition pharmaceutique comprenant: une quantité à effet pharmacologique d'une somatotropine modifiée ou d'un dérivé de somatotropine selon l'une quelconque des revendications 1 à 3 ou des sels pharmaceutiquement acceptables de celle-ci; et un véhicule pharmaceutiquement acceptable, solide ou liquide, pour celle-ci, où ladite composition est efficace pour augmenter la vitesse de croissance d'un animal sur une période prolongée.

5. Composition selon la revendication 4, dans laquelle ladite composition est administrée par voie parentérale audit animal.

### Revendications pour les Etats contractants suivants : ES, GR

1. Procédé de préparation d'un dérivé de somatotropine, dans lequel au moins un résidu de cystéine de ladite somatotropine est modifié avec des substituants, lesdits substituants comprenant: ($CH_2CO_2R_1$), ($CH_2CONHR_1$), [$CH(COR_2)$ $(CH_2)_x(COR_3)$], [$CH_2CONHCH(COR_2)$ - $(CH_2)_x(COR_3)$] ou [$CH_2CONH$-$(CH_2)_xCOR_4$]; dans lesquels $R_1$ est $CH_2CH_2(OCH_2CH_2)_yOMe$; $R_2$ et $R_3$ sont H ou $R_4$; $R_4$ est $NHCH_2CH_2(OCH_2CH_2)_yOMe$ ou $OCH_2CH_2(OCH_2CH_2)_yOMe$; où x est un entier compris entre 1 et 3 et y est un entier compris entre 10 et 300; à condition

que $R_2$ et $R_3$ ne puissent pas être simultanément ; pourvu que, lorsque le(s) résidu(s) de la cystéine modifiée de ladite somatotropine forme(nt) une liaison disulfure avant la modification, les deux cystéines portent le même dérivé sulfhydryle; dans lequel un agent modificateur portant des résidus substituants choisis parmi ($CH_2CO_2R_1$), ($CH_2CONHR_1$), [$CH(COR_2)$ $(CH_2)_x(COR_3)$], [$CH_2CONHCH$ $(COR_2)$ $(CH_2)_x(COR_3)$] ou [$CH_2CONH(CH_2)_xCOR_4$]; dans lesquels $R_1$ est $CH_2CH_2(OCH_2CH_2)_yOMe$; $R_2$ et $R_3$ sont H ou $R_4$; $R_4$ est $NHCH_2CH_2(OCH_2CH_2)_yOMe$ ou $OCH_2CH_2(OCH_2CH_2)_yOMe$; x est un entier compris entre 1 et 3 et y est un entier compris entre 10 et 300; à condition que $R_2$ et $R_3$ ne puissent pas être simultanément H; est mis en réaction avec ladite somatotropine; à conditions que lorsque le(s) résidu(s) de la cystéine modifiée de ladite somatotropine forment une liaison disulfure avant la modification, ladite liaison disulfure soit brisée et réduite avant la modification

**2.** Procédé selon la revendication 1, dans lequel ladite somatotropine contient au moins un résidu de cystéine, sous sa forme naturelle.

**3.** Procédé selon la revendication 1, dans lequel ladite somatotropine a subi l'addition ou la substitution d'un résidu de cystéine, sur la forme naturelle de la somatotropine.

**4.** Procédé de fabrication d'une composition pharmaceutique dans lequel une quantité à effet pharmacologique d'une somatotropine modifiée ou d'un dérivé de somatotropine selon l'une quelconque des revendications 1 à 3 ou des sels pharmaceutiquement acceptables de celle-ci; et un véhicule pharmaceutiquement acceptable, solide ou liquide, pour celle-ci sont formulés, dans lequel ladite composition est efficace pour augmenter la vitesse de croissance d'un animal sur une période prolongée.

**5.** Procédé selon la revendication 4, dans lequel ladite composition est administrée par voie parentérale audit animal.